# EUROPEAN PATENT APPLICATION

(11) **EP 4 596 568 A1**
(43) Date of publication of application: **06.08.2025**
(21) Application number: 23911832.6
(22) Date of filing: 18.12.2023
(51) Int. Cl.: C07K 1/10, C07K 1/02, C07K 1/04, C07K 1/06

(54) **PEPTIDE SYNTHESIS METHOD**

(30) Priority: 26.12.2022 JP 2022208765
(71) Applicant: Kobe Gakuin Educational Foundation, Kobe-shi, Hyogo 650-8586 (JP)
(72) Inventor: HOJO, Keiko, Kobe-shi, Hyogo 650-8586 (JP); TSUDA, Yuko, Kobe-shi, Hyogo 650-8586 (JP)
(74) Representative: Meissner Bolte Partnerschaft mbB
(86) International application number: PCT/JP2023/045375
(87) International publication number: WO 2024/143052

(57) **Abstract**

Provided is a peptide synthesis method that is simpler, more efficient, and/or has a lower environmental impact. The peptide synthesis method includes bringing a mixed dispersion of a protected amino acid for peptide synthesis, a base, a water-soluble condensing agent, and an aqueous solvent into contact with an amino acid and/or a peptide to allow a condensation reaction between the protected amino acid for peptide synthesis and the amino acid and/or the peptide.

## Description

### Technical Field

The present invention relates to a peptide synthesis method and the like.

### Background Art

Liquid-phase synthesis and solid-phase synthesis are known as chemical synthesis methods for peptides. The solid-phase peptide synthesis method was developed by Merrifield in 1963 (see, for example, NPL 1) and is now performed as a standard technique in chemical synthesis of peptides, which has also been automated. Meanwhile, bioactive low-molecular-weight peptides have been recently used in drug discovery, epitope mapping, antibody preparation, and other applications, leading to a growing demand for these molecules. In such circumstances, with the spread of combinatorial chemistry also contributing, solid-phase peptide synthesis methods that can be automated are gaining increasing attention as a means to synthesize peptides more accurately and more rapidly (see, for example, PTL 1 and 2).

The general process of solid-phase peptide synthesis methods is as follows. Amino acids with their N-terminus protected by a protecting group (typically Fmoc or Boc), referred to as "protected amino acids," are coupled at their C-terminus to a solid insoluble support (typically polystyrene resin beads) via a linker. Next, unreacted protected amino acids (i.e.**,** protected amino acids not bound to the support) are removed. Subsequently, the protecting groups of the protected amino acids are removed under conditions in which the protected amino acids bound to the support do not detach from the support. Separately, second protected amino acids, with their N-terminus protected by a protecting group, intended to be coupled with the amino acids bound to the support, are prepared. The second protected amino acids are added to the amino acids bound to the support to allow the N-terminus of the amino acids bound to the support to be condensed with the C-terminus of the second protected amino acids. Next, unreacted second protected amino acids are removed, and then the protecting groups of the second protected amino acids are removed. Then, third amino acids with their N-terminus protected by a protecting group are added, and the N-terminus of the second amino acids is condensed with the C-terminus of the third protected amino acids. This operation is repeated to synthesize a peptide having a desired amino acid sequence bound to a support. The desired peptide is obtained by separating this peptide from the support.

However, many solid-phase peptide synthesis methods consume large amounts of organic solvents because they are used not only as reaction solvents but also repeatedly used in processes such as washing. This is also the case in liquid-phase peptide synthesis methods, which have consumed large amounts of organic solvents. In today's world, in which harmony with the environment is desired, there has been demand for reducing the consumption of organic solvents and using environmentally friendly alternative solvents (e.g., water) even in solid-phase peptide synthesis methods from a perspective of environmental impact. For this reason, attempts are being made to use aqueous solutions in solid-phase peptide synthesis methods.

Leading examples of in-water peptide synthesis techniques include the following: (1) peptide synthesis in water by using water-soluble protected amino acids, (2) peptide synthesis in water by using a surfactant, and (3) peptide synthesis in water by using water-dispersible protected amino acid nanoparticles. However, method (1) requires a separate step for synthesizing protected amino acids, and method (2) requires an additional surfactant, while method (3) requires specialized equipment for preparing nanoparticles. Thus, all of these methods have faced significant barriers to general applicability and automated synthesis.

### Citation List

### Patent Literature

PTL 1: JPH06-220084A
PTL 2: JPH07-089983A

### Non-Patent Literature

NPL 1: R. B. Merrifield, J. Am. Chem. Soc., 85, 2149(1963)

### Summary of Invention

### Technical Problem

An object of the present invention in an aspect is to provide a peptide synthesis method that is simpler, more efficient, and/or has a lower environmental impact.

### Solution to Problem

In view of the object above, the present inventors conducted extensive research and found that the object can be achieved by a peptide synthesis method including bringing a mixed dispersion containing protected amino acids for peptide synthesis, a base, a water-soluble condensing agent, and an aqueous solvent into contact with amino acids and/or peptides to allow a condensation reaction between the protected amino acids for peptide synthesis and the amino acids and/or peptides. The inventors conducted further research based on this finding and completed the present invention. Specifically, the present invention encompasses the following aspects.

### Item 1.

A peptide synthesis method comprising bringing a mixed dispersion of a protected amino acid for peptide synthesis, a base, a water-soluble condensing agent, and an aqueous solvent into contact with an amino acid and/or a peptide to allow a condensation reaction between the protected amino acid for peptide synthesis and the amino acid and/or the peptide.

### Item 2.

The peptide synthesis method according to Item 1, wherein the mixed dispersion contains particles with an average particle size of 1 to 100 nm.

### Item 3.

The peptide synthesis method according to Item 1, wherein the water-soluble condensing agent is at least one selected from the group consisting of a uronium condensing agent and a triazine condensing agent.

### Item 4.

The peptide synthesis method according to Item 1, wherein the water-soluble condensing agent is a salt containing at least one ion selected from the group consisting of BF₄⁻ and Cl⁻.

### Item 5.

The peptide synthesis method according to Item 1, wherein the base is an amine.

### Item 6.

The peptide synthesis method according to Item 1, wherein the base is a tertiary amine.

### Item 7.

The peptide synthesis method according to Item 1, wherein the protected amino acid for peptide synthesis has Fmoc or Boc as a protecting group of the amino group on the main chain.

### Item 8.

The peptide synthesis method according to Item 1, wherein the protected amino acid for peptide synthesis has Fmoc as a protecting group of the amino group on the main chain.

### Item 9.

The peptide synthesis method according to Item 1, wherein the water-soluble condensing agent is a uronium condensing agent, and the protected amino acid for peptide synthesis has Fmoc as a protecting group of the amino group on the main chain.

### Item 10.

The peptide synthesis method according to any one of Items 1 to 9, wherein the content of the base is 0.3 to 5 mol per mole of the protected amino acid for peptide synthesis, and the content of the water-soluble condensing agent is 0.3 to 5 mol per mole of the protected amino acid for peptide synthesis.

### Item 11.

The peptide synthesis method according to any one of Items 1 to 9, wherein the amino acid and/or the peptide is linked to a solid support.

### Item 12.

A mixed dispersion comprising a protected amino acid for peptide synthesis, a base, a water-soluble condensing agent, and an aqueous solvent.

### Item 13.

A reagent for use in the peptide synthesis method of any one of Items 1 to 9, comprising the mixed dispersion of Item 12.

### Advantageous Effects of Invention

The present invention provides a peptide synthesis method that is simpler, more efficient, and/or has a lower environmental impact.

### Brief Description of Drawings

Fig. 1 shows a container filled with water in A, and a container filled with the mixed dispersion of protected amino acids (Fmoc-Phe-OH) prepared in Example 1 in B. These photographic images show the state of the liquid in individual containers irradiated with green laser.
Fig. 2 shows the results of measuring the average particle size of the mixed dispersion of protected amino acids (Fmoc-Phe-OH) prepared in Example 1 according to dynamic light scattering (HORIBA LB-500).
Fig. 3 shows the results of HPLC analysis of the peptide synthesized in Example 1.
Fig. 4 shows the results of HPLC analysis of the peptide synthesized in Example 2.
Fig. 5 shows the results of HPLC analysis of the peptide synthesized in Example 3.
Fig. 6 shows the results of HPLC analysis of the peptide synthesized in Example 4.
Fig. 7 shows the results of HPLC analysis of the peptide synthesized in Example 5.
Fig. 8 shows the results of HPLC analysis of the peptide synthesized in Example 6.
Fig. 9 shows the results of HPLC analysis of the peptide synthesized in Example 7.
Fig. 10 shows the results of HPLC analysis of the peptide synthesized in Example 8. This figure shows the case in which TATU was used as a water-soluble condensing agent.
Fig. 11 shows the results of HPLC analysis of the peptide synthesized in Example 8. This figure shows the case in which TCTU was used as a water-soluble condensing agent.
Fig. 12 shows the results of HPLC analysis of the peptide synthesized in Example 9. The arrow indicates the peak of the target peptide.

### Description of Embodiments

In the present specification, the phrases "comprising" and "containing" include the concepts of comprising, containing, consisting essentially of, and consisting of.

The present invention relates to a peptide synthesis method (which may also be referred to as "the synthesis method of the present invention" in the present specification) comprising bringing a mixed dispersion of a protected amino acid for peptide synthesis, a base, a water-soluble condensing agent, and an aqueous solvent into contact with an amino acid and/or a peptide to allow a condensation reaction between the protected amino acid for peptide synthesis and the amino acid and/or the peptide. The following explains this method.

The protected amino acid for peptide synthesis is a protected amino acid that can be used in peptide synthesis (in particular, solid-phase peptide synthesis), and is not particularly limited as long as the amino group (N-terminal amino group) on the main chain of the amino acid is protected by a protecting group. The protected amino acid for peptide synthesis used in the present invention is a water-insoluble protected amino acid (in particular, an amino acid protected by a hydrophobic protecting group).

The amino acid may be either a natural amino acid or a synthetic amino acid. Examples include amino acids with a basic side chain, such as lysine, arginine, or histidine; amino acids with an acidic side chain, such as aspartic acid or glutamic acid; amino acids with a non-charged polar side chain, such as glycine, asparagine, glutamine, serine, threonine, tyrosine, or cysteine; amino acids with a non-polar side chain, such as alanine, valine, leucine, isoleucine, proline, phenylalanine, methionine, or tryptophan; amino acids with a β-branched side chain, such as threonine, valine, or isoleucine; and amino acids with an aromatic side chain, such as tyrosine, phenylalanine, tryptophan, or histidine. The amino acid can be either D-form or L-form, and can be α-amino acid, β-amino acid, γ-amino acid, or δ-amino acid.

Examples of hydrophobic protecting groups for the amino group on the main chain include 9-fluorenylmethyloxycarbonyl (Fmoc), tert-butoxycarbonyl (Boc), benzyloxycarbonyl (Z), tert-amyloxycarbonyl, isobornyloxycarbonyl, 4-methoxybenzyloxycarbonyl, 2-chlorobenzyloxycarbonyl (Cl-Z), 2-bromobenzyloxycarbonyl (Br-Z), adamantyloxycarbonyl, trifluoroacetyl, phthalyl, formyl, 2-nitrophenylsulfenyl, and diphenylphosphinothioyl. Of these, from the viewpoint of peptide synthesis efficiency, Fmoc and Boc are preferable, and Fmoc is particularly preferable.

In the case of an amino acid with other groups that are not desired to react under condensation reaction conditions (reactive side chains), the amino acid with the reactive side chains protected by appropriate protecting groups can be used. Examples of protecting groups for the carboxyl group include alkyl esters (e.g., ester groups of methyl, ethyl, propyl, butyl, t-butyl, cyclopentyl, cyclohexyl, cycloheptyl, cyclooctyl, or 2-adamantyl), benzyl ester, 2-nitrobenzyl ester, 4-methoxybenzyl ester, 4-chlorobenzyl ester, benzhydryl ester, phenacyl ester, benzyloxycarbonyl hydrazide, t-butoxycarbonyl hydrazide, and trityl hydrazide. The hydroxyl group of serine can be protected, for example, by esterification or etherification. Examples of suitable groups for this esterification include lower alkanoyl groups (preferably alkanoyl groups with 1 to 3 carbon atoms), such as an acetyl group, aroyl groups, such as a benzoyl group, and groups derived from carbonic acid, such as a benzyloxycarbonyl group and an ethoxycarbonyl group. Examples of groups suitable for etherification include a benzyl group, a tetrahydropyranyl group, and a t-butyl group. Examples of protecting groups for the phenolic hydroxyl group of tyrosine include benzyl (Bzl), 2,6-dichlorobenzyl (Cl₂-Bzl), 2-nitrobenzyl, Br-Z, and t-butyl (tBu). Examples of protecting groups for the imidazole of histidine include p-toluenesulfonyl (Tos), 4-methoxy-2,3,6-trimethylbenzenesulfonyl, dinitrophenyl (DNP), benzyloxymethyl, t-butoxymethyl (Bum), Boc, trityl (Trt), and Fmoc.

The protected amino acid for peptide synthesis is typically one type in a mixed dispersion. However, the present disclosure does not exclude a combination two or more types of protected amino acids.

The protected amino acid for peptide synthesis for use may be those known in the art of peptide synthesis (e.g., commercially available protected amino acids), or those designed according to public information and synthesized according to or pursuant to a known method.

The concentration of the protected amino acid for peptide synthesis in the mixed dispersion is preferably 0.1 to 1.0 mol/L, more preferably 0.2 to 0.5 mol/L, and even more preferably 0.20 to 0.30 mol/L, from the viewpoint of its contribution to the formation of the mixed dispersion and peptide synthesis efficiency.

The base contributes to the formation of the mixed dispersion and is not particularly limited. The based for use is preferably an amine. Examples of amines include aliphatic amines (e.g., alkyl amines, more specifically N,N-diisopropylethylamine, trimethylamine, triethylamine, and other trialkyl (e.g., alkyl with 1 to 5 or 1 to 3 carbon atoms) amines, methylamine, ethylamine, dimethylamine, and diethylamine); aromatic amines (e.g., N,N-dialkyl (e.g., alkyl with 1 to 5 or 1 to 3 carbon atoms) aniline, N,N-dialkyl (e.g., alkyl with 1 to 5 or 1 to 3 carbon atoms) phenethylamine, N,N-dialkyl (e.g., alkyl with 1 to 5 or 1 to 3 carbon atoms) toluidine); and heterocyclic amines (e.g., N-alkyl (e.g., alkyl with 1 to 5 or 1 to 3 carbon atoms) morpholine, N-alkyl (e.g., alkyl with 1 to 5 or 1 to 3 carbon atoms) pyrrolidine, N-alkyl (e.g., alkyl with 1 to 5 or 1 to 3 carbon atoms) piperidine, N,N-dialkyl (e.g., alkyl with 1 to 5 or 1 to 3 carbon atoms) piperazine). The amine is particularly preferably a tertiary amine from the viewpoint of its contribution to the formation of the mixed dispersion and peptide synthesis efficiency. More specifically, the tertiary amine is preferably trialkylamine (in particular, N,N-diisopropylethylamine), a heterocyclic amine (in particular, N-alkyl (e.g., alkyl with 1 to 5 or 1 to 3 carbon atoms) morpholine, especially N-methylmorpholine), and more preferably trialkylamine.

The base may be a single type or a combination of two or more types.

The base for use may be a known base (e.g., a commercially available base), or a base designed according to public information and synthesized according to or pursuant to a known method.

The content of the base in the mixed dispersion is preferably 0.3 to 5 mol, more preferably 0.8 to 4 mol, and even more preferably 1.5 to 3 mol per mole of the protected amino acid for peptide synthesis from the viewpoint of its contribution to the formation of the mixed dispersion and peptide synthesis efficiency.

The concentration of the base in the mixed dispersion is preferably 0.1 to 3.0 mol/L, more preferably 0.4 to 1.5 mol/L, even more preferably 0.4 to 1.0 mol/L, and yet more preferably 0.4 to 0.6 mol/L from the viewpoint of its contribution to the formation of the mixed dispersion and peptide synthesis efficiency.

The water-soluble condensing agent contributes to the formation of the mixed dispersion and is not particularly limited. Examples of water-soluble condensing agents include uronium condensing agents (e.g., 1-[bis(dimethylamino)methylene]-1H-benzotriazolium 3-oxide tetrafluoroborate (TBTU), 1-[bis(dimethylamino)methylene]-1H-1,2,3-triazolo[4,5-b]pyridinium 3-oxide tetrafluoroborate (TATU), O-(6-chlorobenzotriazol-1-yl)-N,N,N',N'-tetramethyluronium tetrafluoroborate (TCTU), 1-[bis(dimethylamino)methylene]-5-chloro-1H-benzotriazolium 3-oxide hexafluorophosphate (HCTU), O-benzotriazole-N,N,N',N'-tetramethyluronium hexafluoroborate (HBTU), (1-cyano-2-ethoxy-2-oxoethylideneaminooxy)dimethylamino-morpholino-carbenium hexafluorophosphate (COMU)), triazine condensing agents (e.g., 4-(4,6-dimethoxy-1,3,5-triazin-2-yl)-4-methylmorpholinium chloride (DMT-MM), (4,6-dimethoxy-1,3,5-triazin-2-yl)-(2-octoxy-2-oxoethyl)dimethylammonium trifluoromethanesulfonate (interface-accumulated DMT-MM)), carbodiimide condensing agents (e.g., N,N'-dicyclohexylcarbodiimide (DCC), N,N'-diisopropylcarbodiimide, 1-ethyl-3-dimethylaminopropylcarbodiimide hydrochloride (EDCI)), chloroformate condensing agents (e.g., ethyl chloroformate, and isobutyl chloroformate), acid halide condensing agents (e.g., pivaloyl chloride), imidazole condensing agents (e.g., 1,1'-carbonyldiimidazole (CDI)), and phosphonium condensing agents (e.g., (benzotriazol-1-yloxy)tripyrrolidinophosphonium hexafluorophosphate (PyBOP (trademark)), and bromotripyrrolidinophosphonium hexafluorophosphate (PyBrop (trademark))). Of these, the water-soluble condensing agent is preferably a uronium condensing agent (in particular, TBTU, TATU, and TCTU) and a triazine condensing agent (in particular, DMT-MM), and more preferably a uronium condensing agent from the viewpoint of their contribution to the formation of the mixed dispersion and peptide synthesis efficiency. Additionally, from the same viewpoint, the water-soluble condensing agent is preferably a salt containing at least one ion selected from the group consisting of BF₄⁻ and Cl⁻, and more preferably a salt containing BF₄⁻.

The water-soluble condensing agent may be a single type or a combination of two or more types.

The water-soluble condensing agent for use may be a known water-soluble condensing agent (e.g., a commercially available water-soluble condensing agent), or a water-soluble condensing agent designed according to public information and synthesized according to or pursuant to a known method.

The content of the water-soluble condensing agent in the mixed dispersion is preferably 0.3 to 5 mol, more preferably 0.8 to 4 mol, and even more preferably 1.5 to 3 mol per mole of the protected amino acid for peptide synthesis from the viewpoint of its contribution to the formation of the mixed dispersion and peptide synthesis efficiency.

The concentration of the water-soluble condensing agent in the mixed dispersion is preferably 0.1 to 2.0 mol/L, more preferably 0.2 to 1.0 mol/L, and even more preferably 0.2 to 0.5 mol/L from the viewpoint of its contribution to the formation of the mixed dispersion and peptide synthesis efficiency.

The solvent of the mixed dispersion is an aqueous solvent. The aqueous solvent is a liquid that is water or a mixture of a water-miscible non-aqueous solvent and water in which water accounts for 50 mass% or more, preferably 80 mass% or more, more preferably 90 mass% or more, even more preferably 95 mass% or more, and yet more preferably 99 mass% or more. The non-aqueous solvent includes lower alcohols (e.g., methanol and ethanol) and solvents that are used in condensation reaction for peptide synthesis and that are miscible with water. When environmental impact is considered, it is preferable to avoid using non-aqueous solvents.

In the present invention, the mixed dispersion can be prepared without adding a surfactant. From this viewpoint, the content of the surfactant in the mixed dispersion is, for example, 1 mass% or less, preferably 0.1 mass% or less, more preferably 0.01 mass% or less, even more preferably 0.001 mass% or less, and particularly preferably 0 mass% (containing no surfactant).

Although the mixed dispersion may contain components other than the components described above (the protected amino acid for peptide synthesis, base, water-soluble condensing agent, and aqueous solvent), it is preferred that their content is minimal (or that the mixed dispersion contains no other components). The content of other components in the mixed dispersion is, for example, 10 mass% or less, preferably 5 mass% or less, more preferably 2 mass% or less, even more preferably 1 mass% or less, and particularly preferably 0.1 mass% or less.

The mixed dispersion contains particles. Without wishing to be interpreted restrictively, the particles are those containing the protected amino acid (preferably those containing the protected amino acid, base, and water-soluble condensing agent) or those containing a composite of these components. In an embodiment, the particles can be micelles. The average particle size of the particles (as measured by dynamic light scattering) is preferably 1 to 100 nm, more preferably 5 to 70 nm, even more preferably 10 to 50 nm, and yet more preferably 15 to 40 nm from the viewpoint of peptide synthesis efficiency.

As described above, the mixed dispersion is typically transparent, preferably clear, because the water-insoluble protected amino acid form particles of very small size (e.g., micelles).

The mixed dispersion can be prepared by mixing the components (the protected amino acid for peptide synthesis, base, water-soluble condensing agent, and aqueous solvent). From the viewpoint of efficiency in preparing the mixed dispersion and in peptide synthesis, it is preferable to suspend a protected amino acid for peptide synthesis and a base in an aqueous solvent and then add a water-soluble condensing agent thereto to mix them. Examples of specific operations for suspension include, but are not particularly limited to, stirring (e.g., vortex) and ultrasonic irradiation. The specific operation for mixing components is not particularly limited as long as it is a gentle mixing operation. The temperature during the preparation of the mixed dispersion is, for example, about 10 to 30°C, and preferably about 15 to 25°C.

In the synthesis method of the present invention, the mixed dispersion is brought into contact with an amino acid and/or a peptide to allow a condensation reaction between the carboxyl group of the protected amino acid for peptide synthesis or its active ester and the amino group of the amino acid and/or the peptide.

The amino acid and/or peptide is the target for adding a protected amino acid for peptide synthesis. The amino acid is as described above. The peptide is not particularly limited as long as the peptide is of an amino acid residue number that can be synthesized through peptide chemical synthesis. For example, the peptide can be of 200 amino acid residues or less, 100 amino acid residues or less, 50 amino acid residues or less, 20 amino acid residues or less, or 10 amino acid residues or less. The peptide can also be of, for example, 5 amino acid residues or more, 10 amino acid residues or more, 15 amino acid residues or more, 20 amino acid residues or more, 25 amino acid residues or more, or 28 amino acid residues or more. The synthesis method of the present invention enables efficient synthesis of even longer peptides. The upper limits and the lower limits above can be combined as appropriate. The amino acid and/or peptide has the N-terminal amino group unprotected, thus enabling a condensation reaction with the protected amino acid for peptide synthesis. Additionally, it is desirable that any group (reactive side chain) that is not intended to react under condensation reaction conditions in the amino acid and/or the peptide is protected by an appropriate protecting group.

It is preferable that the amino acid and/or the peptide is bound to a solid support. The solid support for use is not particularly limited, and a wide range of water-insoluble solid supports known in solid-phase peptide synthesis can be used. Some supports have a linker that connects itself with a reaction site in condensation reaction. These supports are also usable and can be appropriately selected according to synthesis conditions. Examples of solid supports include, but are not limited to, styrene resin, acrylamide resin, polyethylene glycol-acrylamide composite resin, and polyoxyethylene-grafted styrene resin. These resins may be crosslinked with divinylbenzene and may be used alone or in a combination of two or more types. Preferably, the solid support is a resin that swells in an aqueous liquid, such as a polyethylene glycol-grafted resin. The linker for use can be selected from a wide range of known linkers. Examples of linkers include, but are not limited to, chloromethyl, hydroxymethyl, benzhydrylamine, aminomethyl, 4-benzyloxybenzyl alcohol, 4-methylbenzhydrylamine, phenylacetamidomethyl, 4-hydroxymethylphenylacetamidomethyl, 4-(2',4'-dimethoxyphenyl-hydroxymethyl) phenoxy, 2-chlorotrityl chloride resin, 4-hydroxymethylphenoxyacetic acid (HMPA), 4-hydroxymethylbenzoic acid (HMBA), and 2,4-dimethoxy-4-hydroxybenzophenone. These may be used alone or in a combination of two or more types.

The reaction temperature for condensation reaction can be appropriately selected from a range known to be usable for peptide bond formation reaction, and may be, for example, room temperature to 80°C, and preferably 50 to 75°C. The pH of the reaction solution is typically 5 to 8, and preferably 6 to 7. The amount of the protected amino acid for peptide synthesis is typically one mol equivalent or more, preferably 3 to 4 mol equivalents, per reaction site (i.e., the amino group) of the solid support or the amino acid and/or the peptide bound to the solid support. If condensation is insufficient (which can be confirmed by, for example, the ninhydrin reaction), condensation reaction with the same protected amino acid for peptide synthesis can be repeated without removing the protecting group to achieve sufficient condensation. When sufficient condensation cannot be achieved even after repeated reaction, unreacted amino acids or unreacted peptides can be acetylated using acetic anhydride or acetyl imidazole.

After condensation reaction, the reaction solution is removed by suction filtration or similar methods, and then the solid support having the protected amino acid for peptide synthesis bound is optionally washed with water, 50% aqueous ethanol, etc.

The optionally washed, solid support having the protected amino acid for peptide synthesis bound is then subjected to a step of removing the protecting group. The step of removing the protecting group can be performed according to a method known in peptide synthesis. A protecting group remover can be appropriately selected considering the protecting group, solid support, and linker used. For example, a solution of 20% v/v piperidine in N,N-dimethylformamide (DMF) can be used for the removal of Fmoc. Moreover, to decrease the amount of the organic solvent used in this step from the viewpoint of reducing the environmental impact, an aqueous protecting group remover, such as sodium hydroxide-containing aqueous ethanol **(e.g.,** 0.1M NaOH-containing 90% aqueous ethanol) can be used. Other protecting group removers include a solution of 25% v/v hydrogen bromide in acetic acid, anhydrous hydrogen fluoride, methanesulfonic acid, trifluoromethanesulfonic acid, and trifluoroacetic acid (TFA). The 2,4-dinitrophenyl group used as a protecting group for the imidazole of histidine can be removed by treatment with thiophenol. The formyl group used as a protecting group for the indole of tryptophan can be removed not only by acid treatment in the presence of 1,2-ethanedithiol or 1,4-butanedithiol described above, but also by alkaline treatment with dilute sodium hydroxide or dilute ammonia. Additionally, the protection and protecting groups for functional groups that should not be involved in the condensation reaction, removal of these protecting groups, activation of functional groups involved in the reaction, and reaction conditions can also be appropriately selected from known groups or known methods.

In solid-phase peptide synthesis, the solid support having a peptide bound, obtained through the step of removing the protecting group, is repeatedly subjected to the condensation reaction and the step of removing the protecting group until the support having a target peptide bound is obtained. Once a solid support having a target peptide bound is obtained, the solid support is subjected to a step of separating the solid support and separated from the target peptide. The step of separating the solid support can be performed according to a method known in solid-phase peptide synthesis, taking into consideration factors such as the presence of protecting groups that should not be involved in the condensation reaction for peptides, the type of the solid support, and the type of the linker. Among methods for the step of removing protecting groups, some methods can remove the protecting group while simultaneously separating the peptide from the solid support. Thus, the step of separating the target peptide from the solid support can be performed concurrently with the step of removing the protecting group in some cases. The separated peptide is optionally purified according to a commonly used method, such as extraction, partitioning, reprecipitation, recrystallization, column chromatography, or high-performance liquid chromatography.

The synthesis method of the present invention allows for the synthesis of a peptide in a simpler, more efficient, and/or more environmentally friendly manner. There are no particular limitations on the target peptide that is synthesized according to the synthesis method of the present invention. The synthesis method of the present invention enables the simple synthesis of various peptides with higher purity in an aqueous solvent while using a commonly used water-insoluble protected amino acid.

### Examples

The following is a detailed explanation of the present invention based on Examples. However, the present invention is not limited to these Examples. Amino acid types are also represented using either a single-letter code or a three-letter code. The term "equivalent" means molar equivalent. All of the mixed dispersions were prepared at room temperature (about 15 to 25°C).

### Example 1: Semi-automated In-water Solid-phase Synthesis of

### Leucine Enkephalin Using Fmoc-protected Amino Acid

Two equivalents of 4-methylmorpholine (NMM), which is a base, were added to one equivalent of an Fmoc-protected amino acid (Fmoc-Phe-OH (product code: K00452), Fmoc-Tyr(tBu)-OH (product code: K00467), Fmoc-Gly-OH (product code: K00434), or Fmoc-Leu-OH (product code: K00440), all manufactured by Watanabe Chemical Industries, Ltd.) and suspended in water by using a vortex. Then, one equivalent of 4-(4,6-dimethoxy-1,3,5-triazin-2-yl)-4-methylmorpholinium chloride (DMT-MM, manufactured by Watanabe Chemical Industries, Ltd.), which is a water-soluble condensing agent, was added thereto and gently mixed to prepare a mixed dispersion. The concentration of each component in the mixed dispersion was as follows: Fmoc-protected amino acid: 0.25 mol/L, base: 0.5 mol/L, water-soluble condensing agent: 0.25 mol/L.

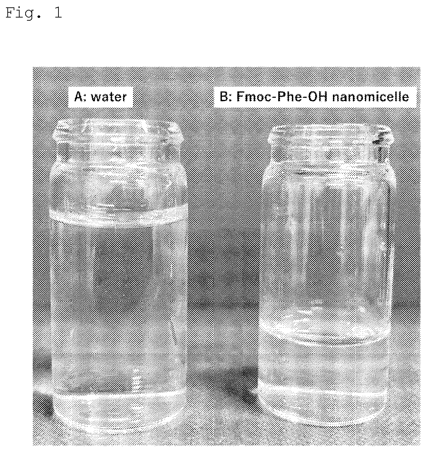

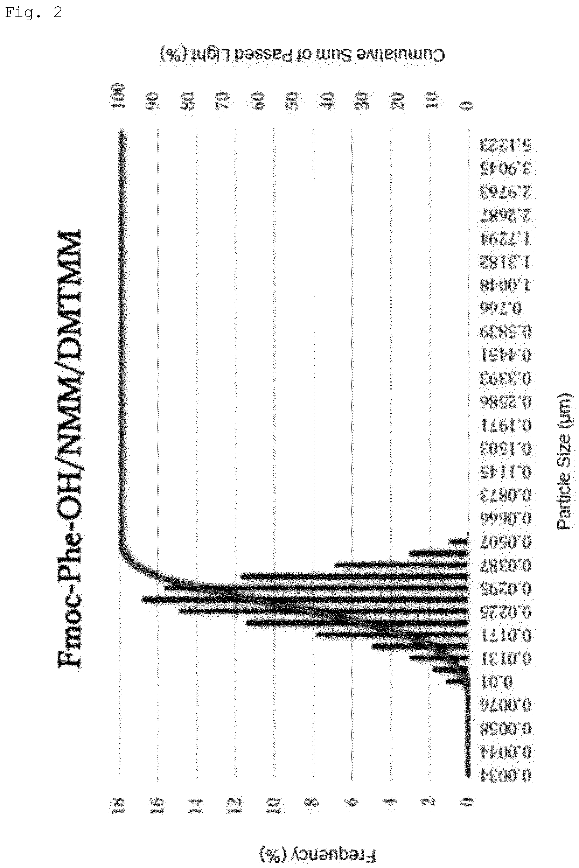

The mixed dispersion was clear. When the mixed dispersion was irradiated with laser light, the Tyndall effect was observed (Fig. 1). The average particle size of the mixed dispersion as measured using dynamic light scattering (HORIBA LB-500) was found to be 20 to 30 nm (Fig. 2). Similar results were obtained for the clarity and particle size of the mixed dispersions in other Examples described below. These results suggested that the mixed dispersion contained particles (in the form of micelles in an embodiment) including an Fmoc-protected amino acid (particles presumably formed of the Fmoc-protected amino acid, base, and water-soluble condensing agent), and that the particles were dispersed, thereby forming a clear liquid.

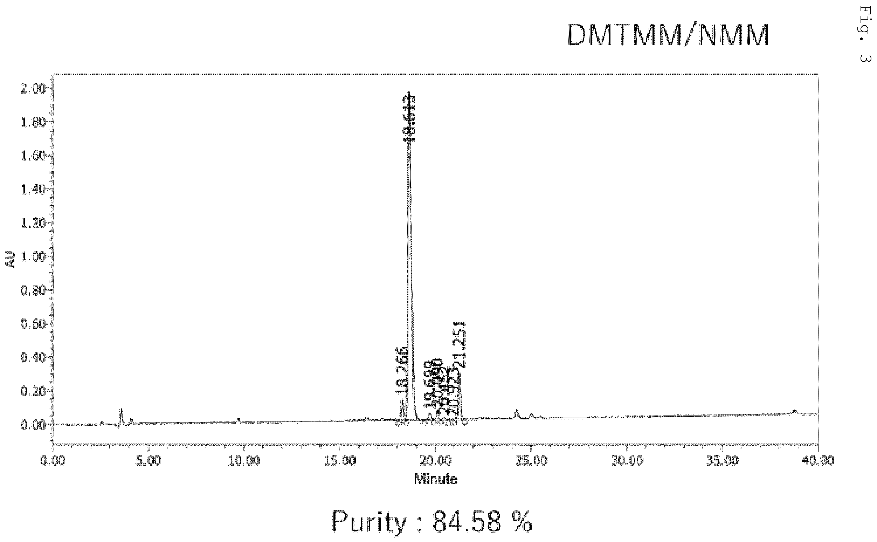

The prepared mixed dispersion was used as is in in-water solid-phase synthesis of leucine enkephalin (YGGFL: SEQ ID NO: 1) using a semi-automatic microwave-assisted synthesizer (Biotage SP Wave) according to the program shown in Table 1. Fmoc-Rinkamide-TantaGel resin was used for the solid phase. After the synthesis was completed, the peptide was cleaved from the resin by treating it with a trifluoroacetic acid cocktail to obtain a crude peptide. The results of HPLC analysis (Waters Alliance system) revealed that the peptide was synthesized with high purity in water (Fig. 3).

**Table 1**

| | **Operation** | **Reagent** | **Temp** | **Time** | **Vortex rate** |
|---|---|---|---|---|---|
| **Wash** | Wash | Water | rt | 1 min x 4 | 900 rpm |
| **Coupling** | Reaction | Fmoc-amino acids nano micelle | 75 °C | 10 min | 900 rpm |
| | Wash | Water | rt | 1 min x 4 | 900 rpm |
| **Wash** | Wash | EtOAc | rt | 1 min x 2 | 900 rpm |
| **Deprotection** | Reation | 20% Piperidine in EtOAc | rt | 10 min x 1 | 900 rpm |
| | Wash | EtOAc | rt | 1 min x 4 | 900 rpm |

### Example 2: Semi-automatic In-water Solid-phase Synthesis of Dermorphin Using Fmoc-protected Amino Acid

Two equivalents of N,N-diisopropylethylamine (DIEA), which is a base, were added to one equivalent of an Fmoc-protected amino acid (Fmoc-Tyr(tBu)-OH (product code: K00467), Fmoc-D-Ala-OH (product code: K00409), Fmoc-Phe-OH (product code: K00452), Fmoc-Gly-OH (product code: K00434), Fmoc-Pro-OH (product code: K00454), or Fmoc-Ser(tBu)-OH (product code: K00458), all manufactured by Watanabe Chemical Industries, Ltd.) and suspended in water by using a vortex. Then, two equivalents of 1-[bis(dimethylamino)methylene]-1H-benzotriazolium 3-oxide tetrafluoroborate (TBTU, manufactured by Tokyo Chemical Industry Co., Ltd.), which is a water-soluble condensing agent, were added thereto and gently mixed to prepare a mixed dispersion. The concentration of each component in the mixed dispersion was as follows: Fmoc-protected amino acid: 0.25 mol/L, base: 0.50 mol/L, water-soluble condensing agent: 0.25 mol/L.

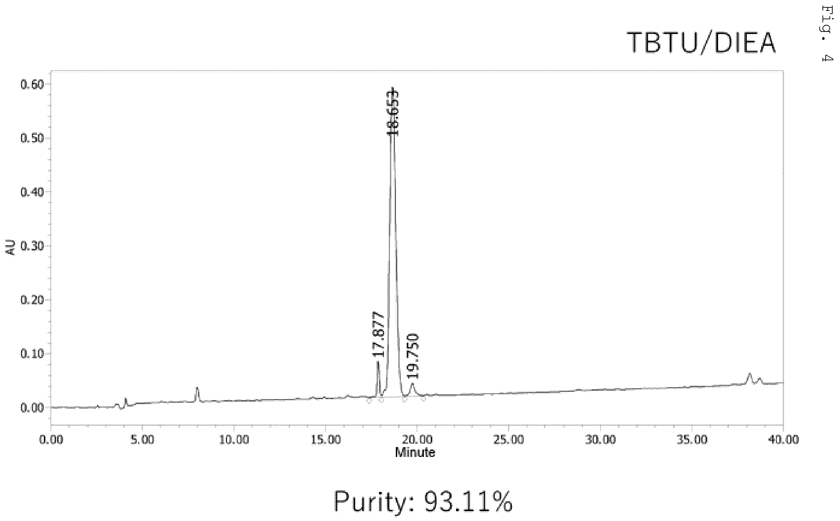

The prepared mixed dispersion was used as is to synthesize dermorphin (Y_{D}AFGYPS: SEQ ID NO: 2) in the same manner as in Example 1. The results of HPLC analysis (Waters Alliance system) revealed that the peptide was synthesized with high purity in water (Fig. 4).

### Example 3: Semi-automated In-water Solid-phase Synthesis of Endogenous Opioid Peptide Using Fmoc-protected Amino Acid

Three equivalents of 4-methylmorpholine (NMM), which is a base, were added to one equivalent of an Fmoc-protected amino acid (Fmoc-Phe-OH (product code: K00452), Fmoc-Tyr(tBu)-OH (product code: K00467), Fmoc-Gly-OH (product code: K00434), or Fmoc-Leu-OH (product code: K00440), all manufactured by Watanabe Chemical Industries, Ltd.) and suspended in water by using a vortex. One equivalent of 4-(4,6-dimethoxy-1,3,5-triazin-2-yl)-4-methylmorpholinium chloride (DMT-MM, manufactured by Watanabe Chemical Industries, Ltd.), which is a water-soluble condensing agent, was added thereto and gently mixed to prepare a mixed dispersion. The concentration of each component in the mixed dispersion was as follows: Fmoc-protected amino acid: 0.25 mol/L, base: 0.75 mol/L, water-soluble condensing agent: 0.25 mol/L.

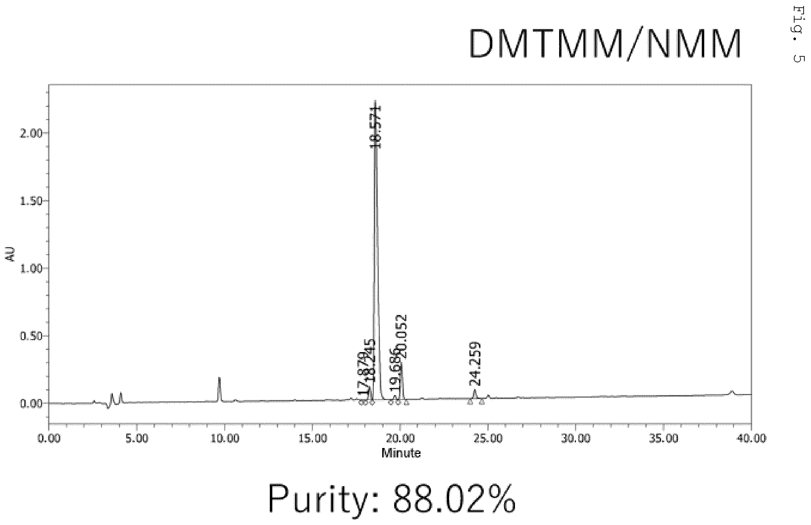

The prepared mixed dispersion was used as is to synthesize an endogenous opioid peptide (YGGFL: SEQ ID NO: 1) in the same manner as in Example 1. The results of HPLC analysis (Waters Alliance system) revealed that the peptide was synthesized with high purity in water (Fig. 5).

### Example 4: Semi-automated In-water Solid-phase Synthesis of Endogenous Opioid Peptide Using Fmoc-protected Amino Acid

Two equivalents of N,N-diisopropylethylamine (DIEA), which is a base, were added to one equivalent of an Fmoc-protected amino acid (Fmoc-Phe-OH (product code: K00452), Fmoc-Tyr(tBu)-OH (product code: K00467), Fmoc-Gly-OH (product code: K00434), or Fmoc-Leu-OH (product code: K00440), all manufactured by Watanabe Chemical Industries, Ltd.) and suspended in water by using a vortex. Then, one equivalent of 1-[bis(dimethylamino)methylene]-1H-benzotriazolium 3-oxide tetrafluoroborate (TBTU, Tokyo Chemical Industry Co., Ltd.), which is a water-soluble condensing agent, was added thereto and gently mixed to prepare a mixed dispersion. The concentration of each component in the mixed dispersion was as follows: Fmoc-protected amino acid: 0.25 mol/L, base: 0.50 mol/L, water-soluble condensing agent: 0.25 mol/L.

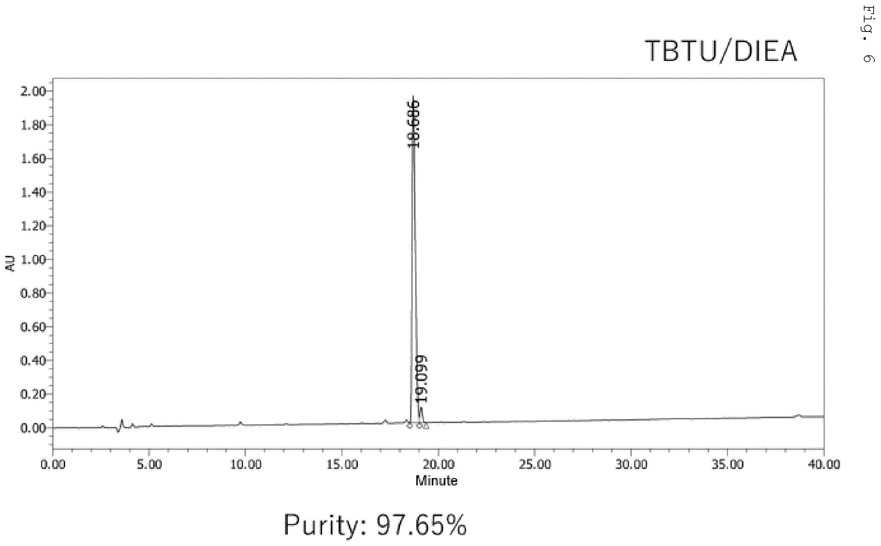

The prepared mixed dispersion was used as is to synthesize an endogenous opioid peptide (YGGFL: SEQ ID NO: 1) in the same manner as in Example 1. The results of HPLC analysis (Waters Alliance system) revealed that the peptide was synthesized with high purity in water (Fig. 6).

### Example 5: Semi-automated In-water Solid-phase Synthesis of Cell Adhesion-related Peptide Using Fmoc-protected Amino Acid

Two equivalents of N,N-diisopropylethylamine (DIEA), which is a base, were added to one equivalent of an Fmoc-protected amino acid (Fmoc-Tyr(tBu)-OH (product code: K00467), Fmoc-Ile-OH (product code: K00439), Fmoc-Gly-OH (product code: K00434), or Fmoc-Arg(Pbf)-OH (product code: K01274), all manufactured by Watanabe Chemical Industries, Ltd.) and suspended in water by using a vortex. Then, one equivalent of 1-[bis(dimethylamino)methylene]-1H-benzotriazolium 3-oxide tetrafluoroborate (TBTU, manufactured by Tokyo Chemical Industry Co., Ltd.), which is a water-soluble condensing agent, was added thereto and gently mixed to prepare a mixed dispersion. The concentration of each component in the mixed dispersion was as follows: Fmoc-protected amino acid: 0.25 mol/L, base: 0.50 mol/L, water-soluble condensing agent: 0.25 mol/L.

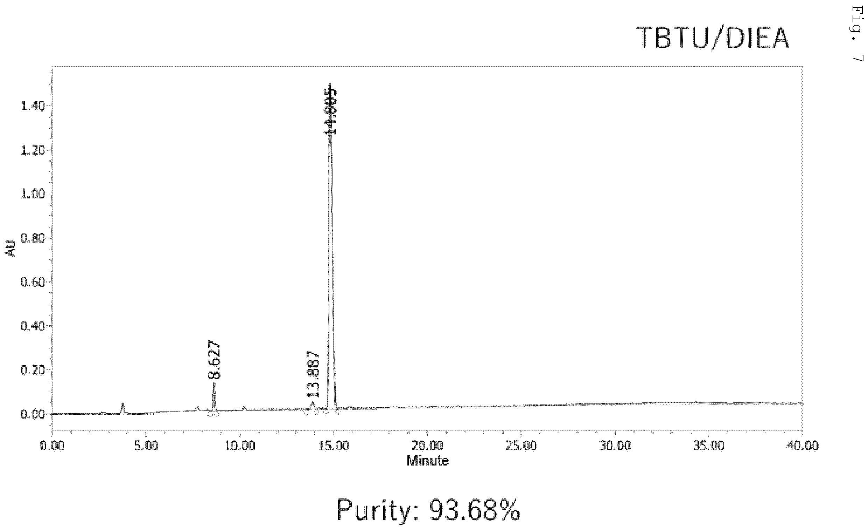

The prepared mixed dispersion was used as is to synthesize a cell adhesion-related peptide (YIGSR: SEQ ID NO: 3) in the same manner as in Example 1. The results of HPLC analysis (Waters Alliance system) revealed that the peptide was synthesized with high purity in water (Fig. 7).

### Example 6: Semi-automated In-water Solid-phase Synthesis of Peptide of Difficult-to-synthesize Sequence Using Fmoc-protected Amino Acid

Two equivalents of N,N-diisopropylethylamine (DIEA), which is a base, were added to one equivalent of an Fmoc-protected amino acid (Fmoc-Val-OH (product code: K00469), Fmoc-Ala-OH (product code: K00408), or Fmoc-Gly-OH (product code: K00434), all manufactured by Watanabe Chemical Industries, Ltd.) and suspended in water by using a vortex. Then, two equivalents of 1-[bis(dimethylamino)methylene]-1H-benzotriazolium 3-oxide tetrafluoroborate (TBTU, manufactured by Tokyo Chemical Industry Co., Ltd.), which is a water-soluble condensing agent, were added thereto and gently mixed to prepare a mixed dispersion. The concentration of each component in the mixed dispersion was as follows: Fmoc-protected amino acid: 0.25 mol/L, base: 0.50 mol/L, water-soluble condensing agent: 0.25 mol/L.

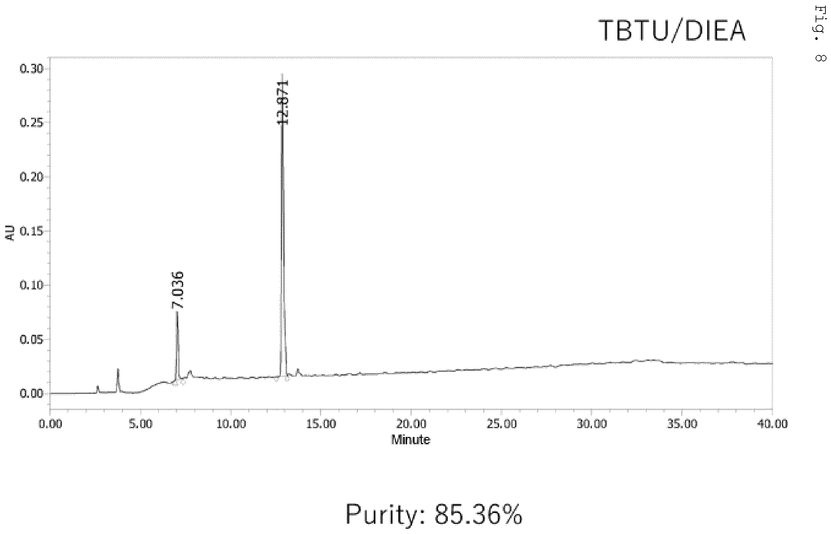

The prepared mixed dispersion was used as is to synthesize a peptide of difficult-to-synthesize sequence (VAVAG: SEQ ID NO: 4) in the same manner as in Example 1. The results of HPLC analysis (Waters Alliance system) revealed that the peptide was synthesized with high purity in water (Fig. 8).

### Example 7: Semi-automated In-water Solid-phase Synthesis of Oxytocin Using Fmoc-protected Amino Acid

Two equivalents of N,N-diisopropylethylamine (DIEA), which is a base, were added to one equivalent of an Fmoc-protected amino acid (Fmoc-Leu-OH (product code: K00440), Fmoc-Pro-OH (product code: K00454), Fmoc-Cys(Acm)-OH (product code: K00424), Fmoc-Asn(Trt)-OH (product code: K00900), Fmoc-Gln(Trt)-OH (product code: K00902), or Fmoc-Tyr(tBu)-OH (product code: K00467), all manufactured by Watanabe Chemical Industries, Ltd.) and suspended in water by using a vortex. Then, one equivalent of 1-[bis(dimethylamino)methylene]-1H-benzotriazolium 3-oxide tetrafluoroborate (TBTU, manufactured by Tokyo Chemical Industry Co., Ltd.), which is a water-soluble condensing agent, was added thereto and gently mixed to prepare a mixed dispersion. The concentration of each component in the mixed dispersion was as follows: Fmoc-protected amino acid: 0.25 mol/L, base: 0.50 mol/L, water-soluble condensing agent: 0.25 mol/L.

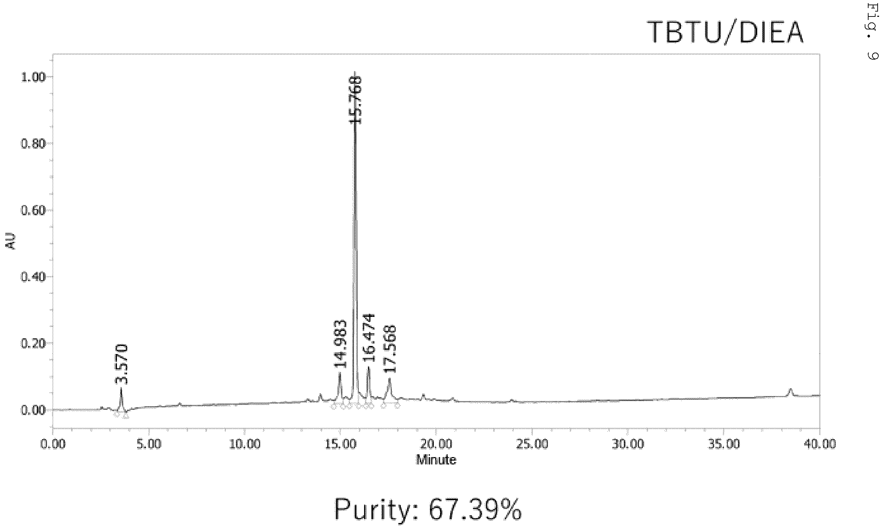

The prepared mixed dispersion was used as is to synthesize oxytocin (CYIQNCPL: SEQ ID NO: 5) in the same manner as in Example 1. The results of HPLC analysis (Waters Alliance system) revealed that the peptide was synthesized with high purity in water (Fig. 9). Although the peptide generally contains Cys residues, which are prone to racemization, the present method resulted in a low rate of racemization.

### Example 8: Semi-automated In-water Solid-phase Synthesis of Peptide Using Fmoc-protected Amino Acid

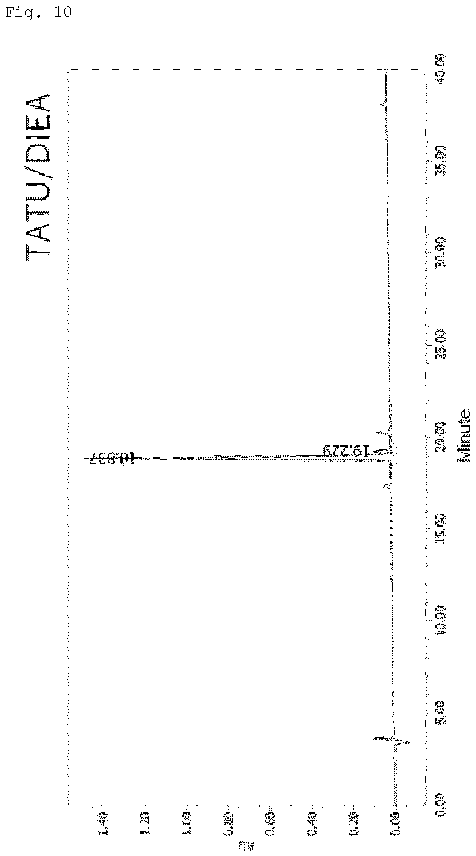

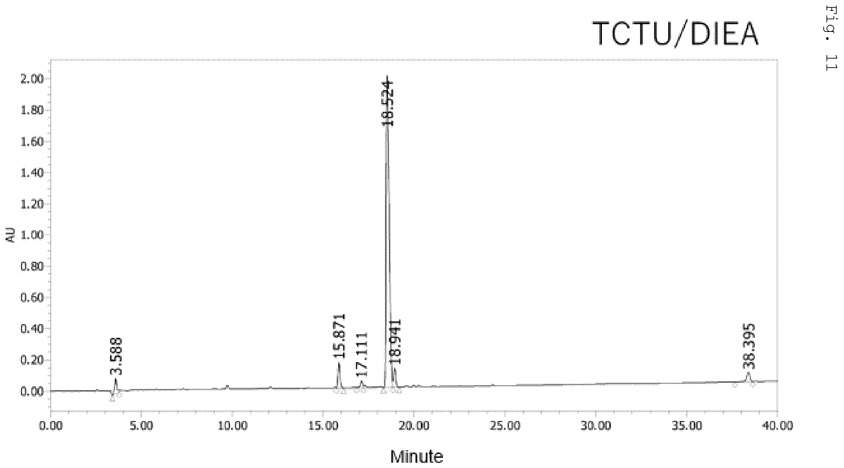

A mixed dispersion was prepared, and then a peptide (YGGFL: SEQ ID NO: 1) was synthesized in the same manner as in Example 2 and other Examples, except for using 1-[bis(dimethylamino)methylene]-1H-1,2,3-triazolo[4,5-b]pyridinium 3-oxide tetrafluoroborate (TATU, manufactured by Tokyo Chemical Industry Co., Ltd.) or O-(6-chlorobenzotriazol-1-yl)-N,N,N',N'-tetramethyluronium tetrafluoroborate (TCTU, manufactured by Tokyo Chemical Industry Co., Ltd.) instead of TBTU. The results of HPLC analysis (Waters Alliance system) revealed that the peptide was synthesized with high purity in water as in Example 2 and other Examples (Figs. 10 and 11).

### Example 9: Semi-automated In-water Solid-phase Synthesis of β-endorphin Analog Using Fmoc-protected Amino Acid

Two equivalents of N,N-diisopropylethylamine (DIEA), which is a base, were added to one equivalent of an Fmoc-protected amino acid (Fmoc-Tyr(tBu)-OH (product code: K00467), Fmoc-Gly-OH (product code: K00434), Fmoc-Phe-OH (product code: K00452), Fmoc-Met-OH (product code: K00446), Fmoc-Ser(tBu)-OH (product code: K00458), Fmoc-Glu(OtBu)-OH (product code: K00428), Fmoc-Lys(Boc)-OH (product code: K00443), Fmoc-Gln(Trt)-OH (product code: K00902), Fmoc-Pro-OH (product code: K00454), Fmoc-Leu-OH (product code: K00440), Fmoc-Val-OH (product code: K00469), Fmoc-Asn(Trt)-OH (product code: K00900), Fmoc-D-Ala-OH (product code: K00409), or Fmoc-Ile-OH (product code: K00439), all manufactured by Watanabe Chemical Industries, Ltd.) and suspended in water by using a vortex. Then, one equivalent of 1-[bis(dimethylamino)methylene]-1H-benzotriazolium 3-oxide tetrafluoroborate (TBTU, manufactured by Tokyo Chemical Industry Co., Ltd.), which is a water-soluble condensing agent, was added thereto and gently mixed to prepare a mixed dispersion. The concentration of each component in the mixed dispersion was as follows: Fmoc-protected amino acid: 0.25 mol/L, base: 0.50 mol/L, water-soluble condensing agent: 0.25 mol/L.

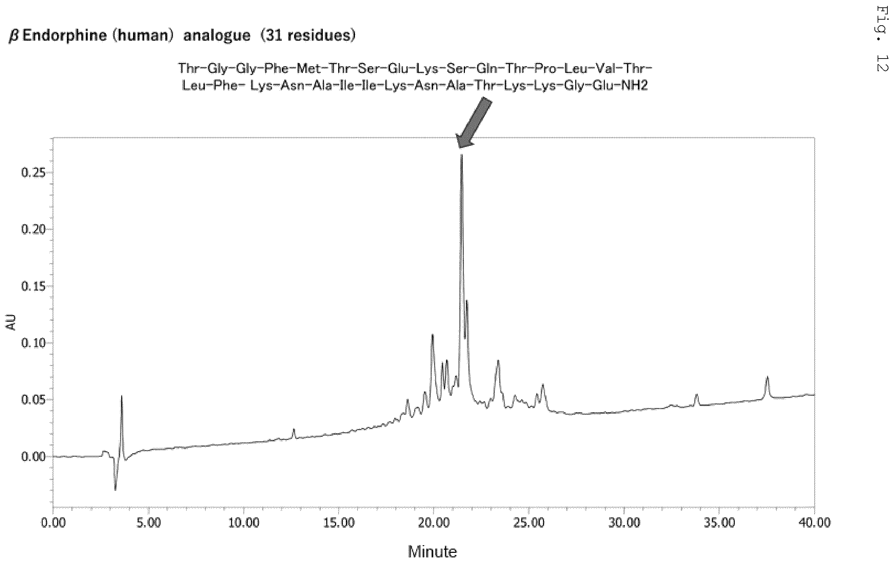

The prepared mixed dispersion was used as is to synthesize a β-endorphin analog (TGGFMTSEKSQTPLVTLFKNAIIKNATKKGE: SEQ ID NO: 6) in the same manner as in Example 1. The results of HPLC analysis (Waters Alliance system) revealed that even a long-chain peptide of 31 residues was able to be synthesized in water with relatively high purity (Fig. 12).

### Sequence Listing

P23-261WO_PCT_peptide synthesis method_20231218_115957_1.xml

## Claims

1. A peptide synthesis method comprising bringing a mixed dispersion of a protected amino acid for peptide synthesis, a base, a water-soluble condensing agent, and an aqueous solvent into contact with an amino acid and/or a peptide to allow a condensation reaction between the protected amino acid for peptide synthesis and the amino acid and/or the peptide.

2. The peptide synthesis method according to claim 1, wherein the mixed dispersion contains particles with an average particle size of 1 to 100 nm.

3. The peptide synthesis method according to claim 1, wherein the water-soluble condensing agent is at least one selected from the group consisting of a uronium condensing agent and a triazine condensing agent.

4. The peptide synthesis method according to claim 1, wherein the water-soluble condensing agent is a salt containing at least one ion selected from the group consisting of BF₄⁻ and Cl⁻.

5. The peptide synthesis method according to claim 1, wherein the base is an amine.

6. The peptide synthesis method according to claim 1, wherein the base is a tertiary amine.

7. The peptide synthesis method according to claim 1, wherein the protected amino acid for peptide synthesis has Fmoc or Boc as a protecting group of the amino group on the main chain.

8. The peptide synthesis method according to claim 1, wherein the protected amino acid for peptide synthesis has Fmoc as a protecting group of the amino group on the main chain.

9. The peptide synthesis method according to claim 1, wherein the water-soluble condensing agent is a uronium condensing agent, and the protected amino acid for peptide synthesis has Fmoc as a protecting group of the amino group on the main chain.

10. The peptide synthesis method according to any one of claims 1 to 9, wherein the content of the base is 0.3 to 5 mol per mole of the protected amino acid for peptide synthesis, and the content of the water-soluble condensing agent is 0.3 to 5 mol per mole of the protected amino acid for peptide synthesis.

11. The peptide synthesis method according to any one of claims 1 to 9, wherein the amino acid and/or the peptide is linked to a solid support.

12. A mixed dispersion comprising a protected amino acid for peptide synthesis, a base, a water-soluble condensing agent, and an aqueous solvent.

13. A reagent for use in the peptide synthesis method of any one of claims 1 to 9, comprising the mixed dispersion of claim 12.
